# EUROPEAN PATENT APPLICATION

(11) **EP 3 900 722 A1**
(43) Date of publication of application: **27.10.2021**
(21) Application number: 20170932.6
(22) Date of filing: 22.04.2020
(51) Int. Cl.: A61K 31/437, A61K 31/4375

(54) **COMBINATION OF FINERENONE AND PECAVAPTAN FOR THE TREATMENT AND/OR PREVENTION OF CARDIOVASCULAR AND/OR RENAL DISEASES**

(71) Applicant: Bayer AG, 51373 Leverkusen (DE)
(72) Inventor: The designation of the inventor has not yet been filed
(74) Representative: BIP Patents

(57) **Abstract**

The present invention relates to pharmaceutical compositions and combinations comprising finerenone or a hydrate, solvate or pharmaceutically acceptable salt thereof or a polymorph thereof and pecavaptan, or a hydrate, solvate or pharmaceutically acceptable salt thereof or a polymorph thereof. The combination can be used for the treatment and/or prevention of cardiovascular and/or renal diseases in humans and other mammals.

## Description

The invention refers to the combination of finerenone or a hydrate, solvate, pharmaceutically acceptable salt thereof, or a polymorph thereof, and pecavaptan or a hydrate, solvate, pharmaceutically acceptable salt thereof, or a polymorph thereof, for the treatment and/or prevention of cardiovascular and/or renal diseases. In particular, these diseases can be characterized by chronic sodium retention.

The liquid content of the human body is subject to various physiological control mechanisms, the purpose of which is to keep it constant (volume homeostasis). In the process, both the volume filling of the vascular system and also the osmolarity of the plasma are continuously recorded by appropriate sensors (baroreceptors and osmoreceptors). The information which these sensors supply to the relevant centers in the brain regulates drinking behavior and controls fluid excretion via the kidneys by means of humoral and neural signals.

The steroid hormone aldosterone plays a key part in maintaining fluid and electrolyte homeostasis by promoting, in the epithelium of the distal nephron, sodium retention and potassium secretion, thus, contributing to keep the extracellular volume constant and, thus, to regulate blood pressure. Besides this, aldosterone displays direct effects on the structure and function of the cardiac and vascular system, but the underlying mechanisms thereof are not yet fully explained (R.E. Booth, J.P. Johnson, J.D. Stockand, Adv. Physiol. Educ. 26 (1), 8-20 (2002)).

Finerenone, which is (S)-4-(4-cyano-2-methoxyphenyl)-5-ethoxy-2,8-dimethyl-1,4-dihydro-1,6-naphthyridin-3-carboxamide, a compound of formula (I) 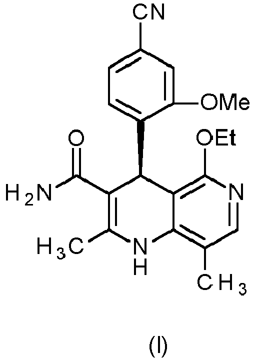 is a mineralocorticoid receptor antagonist (MR antagonist). The synthesis, pharmacological properties and pharmaceutical formulations/dosage forms are described in detail in WO 2008/104306 A1. WO 2008/104306 A1 states that the MR IC50 of 4-(4-cyano-2-methoxyphenyl)-5-ethoxy-2,8-dimethyl-1,4-dihydro-1,6-naphthyridine-3-carboxamide (racemate, Example 4) is 23 nM and the IC50 of *ent*-4-(4-cyano-2-methoxyphenyl)-5-ethoxy-2,8-dimethyl-1,4-dihydro-1,6-naphthyridine-3-carboxamide ((-)-enantiomer, Example 5; "finerenone") is 16 nM (see WO2008/104306 A1, section B.1 "Assessment of the pharmacological activity"). A further process for the manufacturing of finerenone is described in WO 2016/016287 A1. MR antagonists (such as the steroidal compounds spironolactone, canrenone/canrenoate and eplerenone, and also more recent non-steroidal MR antagonists such as MT-3995, CS-3150, AZD9977, KBP-5074, LY2623091, PF-03882845 and finerenone) counteract aldosterone-mediated sodium retention in the kidneys (natriuretic effect). Thus, MR antagonists lead to increased sodium excretion, which is a proven therapeutic concept for hypertensive patients and/or patients suffering from heart failure and/or kidney failure. However, MR antagonists can unfold their natriuretic action only in kidney segments in which aldosterone also exerts its physiological action via the MR. These are in particular the late distal tubulus and collecting duct sections involved in sodium re-resorption only to a limited extent, whereas most of sodium secretion and re-resorption takes place in proximal tubulus sections. In absolute terms, aldosterone only influences about 2% of the total sodium reabsorption via MR in the distal tubule and in the collecting tube, thereby limiting the natriuretic potency of MR antagonists. Although use of MR antagonists is already part of the pharmacological treatment according to guidelines for chronic heart failure patients, the natriuretic potency of MR antagonists is limited.

Also the peptide hormone vasopressin is of central importance in volume homeostasis (Schrier R.W., Abraham W.T., New Engl. J. Med. 341, 577-585 (1999)). Vasopressin exerts its action mainly via binding to three receptors, which are classified as Via, V1b and V2 receptors and which belong to the family of G protein-coupled receptors. Via receptors are mainly located on the cells of the vascular smooth musculature. Their activation gives rise to vasoconstriction, as a result of which the peripheral resistance and blood pressure rise. Apart from this, V1a receptors are also detectable in the liver. V1b receptors (also named V3 receptors) are detectable in the central nervous system. Together with corticotropin-releasing hormone (CRH), vasopressin regulates the basal and stress-induced secretion of adrenocorticotropic hormone (ACTH) via the V1b receptor. V2 receptors are located in the distal tubular epithelium and the epithelium of the collecting tubules in the kidney. Their activation renders these epithelia permeable to water. This phenomenon is due to the incorporation of aquaporins (special water channels) in the luminal membrane of the epithelial cells.

As described in WO 2016071212 A1, agents which inhibit the action of vasopressin on the V2 and/or the V1a receptor appear suitable for the treatment of heart failure. In particular, compounds with combined activity on both vasopressin receptors (V1a and V2) should have both desirable renal as well as hemodynamic effects and thus offer an especially ideal profile for the treatment of patients with heart failure.

Pecavaptan, which is 5-(4-chlorophenyl)-2-({1-(3-chlorophenyl)-5-[(1S)-1-hydroxyethyl]-1H-1,2,4-triazol-3-yl}methyl)-4-[(2S)-3,3,3-trifluoro-2-hydroxypropyl]-2,4-dihydro-3H-1,2,4-triazol-3-one a compound of formula (II) 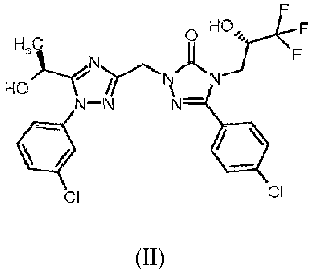 is a dual vasopressin antagonist (V1a and V2). The synthesis, pharmacological properties and pharmaceutical formulations/dosage forms are described in detail in WO 2016/071212 A1. In particular, pecavaptan is a dual V1a/V2 antagonist with an IC50 value of 0.0036 µM at the human V1a receptor and 0.0017 µM at the human V2 receptor (see WO 2016/071212 A1, section B-1, Cellular in vitro assay for determining vasopressin receptor activity, Example 79). Agents which inhibit the action of vasopressin on the V1a and/or the V2 receptor appear suitable for the treatment of heart failure. In particular, compounds with combined activity on both vasopressin receptors (V1a and V2) should have both aquaretic (via V2 receptor blockade) as well as hemodynamic effects (via V1a receptor blockade) and, thus, offer a certain profile for the treatment of patients with heart failure. A volume diminution mediated solely via V2 receptor blockade can entail the stimulation of osmoreceptors and, as a result, may lead to a further compensatory increase in vasopressin release. Through this, in the absence of a component simultaneously blocking the V1a receptor, the harmful effects of vasopressin, such as for example vasoconstriction and cardiac hypertrophy, could be further intensified (Saghi P. et al., Europ. Heart J. 26, 538-543 (2005)).

In summary, in addition to purely water-based volume excretion ('aquaresis'), sodium excretion via the urine ('natriuresis') is a particular therapeutic target for cardiovascular diseases with congestion symptoms. For example, neurohumoral stimulation by the renin-angiotensin-aldosterone system (RAAS) and vasopressin system leads to permanent sodium retention and an associated extracellular volume load in the event of acute and chronic heart failure. The body is no longer able to perform effective natriuresis, especially when there is impaired kidney function, which is often associated with heart failure. Therefore, maintaining an effective natriuresis would be useful in treatment and/or prevention of cardiovascular and/or renal diseases.

An object of the present invention is the improvement of the treatment and/or prevention of cardiovascular and renal diseases in comparison to the already known monotherapies.

A further object of the present invention is to provide combinations of active pharmaceutical ingredients for the treatment of cardiovascular diseases, which are characterized by chronic sodium retention, in particular also cardiac and renal insufficiency, which reduce mortality and / or morbidity in patients.

Another object of the present invention is the improvement of the treatment and/or prevention of cardiovascular and renal diseases by administration of a combination comprising finerenone and pecavaptan.

The present invention pertains to a combination comprising finerenone with pecavaptan.

The invention provides for combinations of active pharmaceutical ingredients, namely the combination of finerenone with pecavaptan, for the treatment of cardiovascular diseases which are characterized by chronic sodium retention or kidney diseases. Cardiovascular diseases are for example congestive heart failure independent of ejection fraction (i.e. with preserved ejection fraction (HFpEF), with mid-range ejection fraction (HFmrEF) or heart failure with reduced ejection fraction (HFrEF)). Renal diseases are for example chronic kidney disease (CKD) and diabetic kidney disease, as well as other syndromes, for example cardiorenal syndrome, nephrotic syndrome, hepatorenal syndrome or end organ damage affecting the heart and kidney, which contribute to an increased mortality and/or morbidity in patients.

Surprisingly the combination of finerenone with pecavaptan shows a significant efficacy improvement over the sum of the monotherapies:
The combination of finerenone with pecavaptan leads to over-additive sodium excretion in comparison to the respective monotherapy with finerenone or pecavaptan alone. This over-additive effect was not predictable and goes beyond the pure addition of the effects of the monotherapies with finerenone and pecavaptan. The over-additive effect achieved with the combination according to the invention was also not predictable as finerenone and pecavaptan target different receptors and/or points of the homeostasis, respectively.

This over-additive sodium excretion effected by the combination according to the invention leads to a subsequent congestion relief since sodium is the most important ion in the body controlling volume homeostasis. A further water excretion takes place through sodium excretion. The water excretion removes unwanted water retention in body tissue. Among other things, there is also a reduction in preload in the heart. The cardiovascular system is relieved. An therapeutic improvement is thus achieved by the combination according to the invention. The combination therapy according to the invention can permanently relieve the overall circulation. This natriuretic efficacy improvement is a major clinical goal in the treatment of cardiovascular and renal diseases.

The combination according to the invention could provide for the administration of lesser amounts of the administered therapeutic agents. The combination according to the invention could provide for a longer survival time among treated patients compared to standard treatments.

The present invention refers to a combination comprising finerenone, which is the compound of the formula (I), or a hydrate, solvate or pharmaceutically acceptable salt of thereof, or a polymorph thereof, and pecavaptan, which is the compound of formula (II), or a hydrate, solvate or pharmaceutically acceptable salt of thereof, or a polymorph thereof.

The "active ingredients" used in the combination according to the invention are finerenone and pecavaptan.

The term "the compound of formula (I)" or "finerenone" refers to ((S)-4-(4-cyano-2-methoxyphenyl)-5-ethoxy-2, 8-dimethyl-1,4-dihydro-1,6-naphthyridin-3 -carboxamide or *ent*-4-(4-cyano-2-methoxyphenyl)-5 - ethoxy-2,8-dimethyl-1,4-dihydro-1,6-naphthyridine-3-carboxamide (WO 2008/104306 A1, Example 5, (-)enantiomer) as depicted in formula (I), or a hydrate, solvate, pharmaceutically acceptable salt thereof, or a polymorph thereof. ((S)-4-(4-cyano-2-methoxyphenyl)-5-ethoxy-2,8-dimethyl-1,4-dihydro-1,6-naphthyridin-3-carboxamide and *ent*-4-(4-cyano-2-methoxyphenyl)-5-ethoxy-2,8-dimethyl-1,4-dihydro-1,6-naphthyridine-3-carboxamide are synonyms. In the following, when referring herein to "finerenone", also a hydrate, solvate, pharmaceutically acceptable salt thereof, or a polymorph thereof is meant. Thus, "finerenone" is sometimes used as synonym for "finerenone, or a hydrate, solvate, pharmaceutically acceptable salt thereof, or a polymorph thereof." According to the invention, finerenone can be used in the combination in form of an hydrate, solvate, pharmaceutically acceptable salt thereof, or a polymorph thereof. A polymorph of finerenone is described in WO201616287A1. The term "finerenone" also comprises the anhydrate thereof.

The term "the compound of formula (II)" or "pecavaptan" refers to 5-(4-chlorophenyl)-2-({1-(3-chlorophenyl)-5-[(1S)-1-hydroxyethyl]-1H-1,2,4-triazol-3-yl}methyl)-4-[(2S)-3,3,3-trifluoro-2-hydroxypropyl]-2,4-dihydro-3H-1,2,4-triazol-3-one, as depicted in formula (II) or a hydrate, solvate, pharmaceutically acceptable salt thereof, or a polymorph thereof. In the following, when referring herein to "pecavaptan", also a hydrate, solvate, pharmaceutically acceptable salt thereof, or a polymorph thereof is meant. Thus, "pecavaptan" is sometimes used as synonym for "pecavaptan, or a hydrate, solvate, pharmaceutically acceptable salt thereof, or a polymorph thereof." According to the invention, pecavaptan can be used in the combination in form of an hydrate, solvate, pharmaceutically acceptable salt thereof, or a polymorph thereof. The term "pecavaptan" also comprises the anhydrate thereof.

"Solvates" for the purposes of the invention are those forms of the compounds or their salts where solvent molecules form a stoichiometric complex in the solid state and include, but are not limited to for example water, ethanol and methanol.

"Hydrates" are a specific form of solvates, where the solvent molecule is water. Hydrates of the compounds of the invention or their salts are stoichiometric compositions of the compounds or salts with water, such as, for example, monohydrate, dihydrates, trihydrate, hemihydrate, sequihydrate.

"Salts" for the purposes of the present invention are preferably "pharmaceutically acceptable salts" of finerenone and/or pecavaptan. Suitable pharmaceutically acceptable salts that can be used in the combination according to the invention are well known to those skilled in the art and include salts of inorganic acids, organic acids, inorganic bases, alkaline cations, alkaline earth cations and organic bases. In one embodiment the pharmaceutically acceptable salt can be selected from hydrochloric acid, hydrobromic acid, sulfuric acid, phosphoric acid, methane sulphonic acid, trifluoromethanesulfonic acid, benzenesulfonic acid, p-toluene sulfonic acid, 1-naphthalenesulfonic acid, 2-naphthalenesulfonic acid, acetic acid, trifluoroacetic acid, malic acid, tartaric acid, citric acid, lactic acid, oxalic acid, succinic acid, fumaric acid, maleic acid, benzoic acid, salicylic acid, phenylacetic acid, and mandelic acid acetate, benzoate, besylate, bromide, camsylate, carbonate, citrate, edisylate, estolate, fumarate, gluceptate, gluconate, glucuronate, hippurate, iodide, isethionate, lactate, lactobionate, malate, maleate, mesylate, methylsulfate, napsylate, nitrate, oxalate, pamoate, phosphate, stearate, succinate, sulfate, tartrate, bitartrate, tosylate, calcium, diolamine, lithium, lysine, magnesium, meglumine, N-methylglucamine, olamine, potassium, tromethamine, tris(hydroxymethyl)aminomethane, benzenesulfonate, ethanesulfonate and zinc.

In one embodiment the pharmaceutically acceptable salt can be selected from hydrochloride, sulfate, mesylate, tosylate, tartrate, citrate, benzenesulfonate, ethanesulfonate, maleate, and phosphate.

The term "combination" in the present invention is used as known to persons skilled in the art. The combination comprises or consists of finerenone or a hydrate, solvate, pharmaceutically acceptable salt thereof, or a polymorph thereof, and pecavaptan or a hydrate, solvate, pharmaceutically acceptable salt thereof, or a polymorph thereof. "Combination" for the purposes of the invention comprises fixed combinations, combination packs, kit-of-parts, non-fixed combinations and/or components (finerenone, pecavaptan), which are administered simultaneously or sequentially. The term "the components" refers to finerenone or a hydrate, solvate, pharmaceutically acceptable salt thereof, or a polymorph thereof, and pecavaptan or a hydrate, solvate, pharmaceutically acceptable salt thereof, i.e. one component of the combination according to the invention is finerenone or a hydrate, solvate, pharmaceutically acceptable salt thereof, or a polymorph thereof and the other component is pecavaptan or a hydrate, solvate, pharmaceutically acceptable salt thereof to. The combination according to the invention can also comprise further components.

In one embodiment according to the invention, the combination is a fixed combination. A "fixed combination" in the present invention is used as known to persons skilled in the art. It is defined as a combination comprising or containing finerenone and pecavaptan within one pharmaceutical dosage form or in one single entity. One example of a "fixed combination" is a pharmaceutical composition, wherein finerenone and pecavaptan are present in admixture. Another example of a "fixed combination" is a pharmaceutical combination, wherein finerenone and pecavaptan are present in one dosage form without being in admixture. Another example of a "fixed combination" is a pharmaceutical combination, wherein finerenone and pecavaptan are present at least partially admixture.

"Pharmaceutical dose form" and "dosage form" are synonyms. "Pharmaceutical dose form" or "dosage form" is the physical manifestation of a product that contains or comprises the active ingredient and/or inactive ingredients (e.g. carrier, excipients) that are intended to be delivered to the patient. "Dosage form" is the term used in the European Pharmacopoeia. "Dosage form" was previously used in Standard Terms, but the term "pharmaceutical dose form" is now used in order to harmonise with the vocabulary that is used across the Identification of Medicinal Products project (cf. https://www.edqm.eu/sites/default/files/standard_terms_introduction and_guidance_for_use.pdf). Common dosage forms include pill, tablet, capsule, syrup, aerosol, liquid injection, powder, or solid crystal, and so on. Further pharmaceutical formulations or dosage forms are disclosed below. The route of administration for drug delivery is dependent on the dosage form of the active ingredient.

In one embodiment according to the invention, the combination is a combined pharmaceutical dose form. The "combined pharmaceutical dose form" is used to combine two or more pharmaceutical dose forms into a single term, in order to describe a medicinal product that consists of two or more manufactured items that are intended to be combined to produce a single pharmaceutical product for administration to the patient. A combined pharmaceutical dose form is not used to combine pharmaceutical dose forms that are packaged together, but administered separately rather than being combined to produce a single pharmaceutical product (see instead combination packs). In one embodiment the combined pharmaceutical dose form comprises a soluble powder, tablet or granulate, which comprises finerenone and/or pecavaptan, and a solvent for solving or dispersing the powder, tablet or granulate. The combined pharmaceutical dose form is not limited to this exemplary embodiment. Various combinations of the dosage forms disclosed herein are suitable for the combination according to the invention.

In one embodiment according to the invention, the combination is a non-fixed combination or kit-of-parts. A "non-fixed combination" or "kit-of-parts" in the present invention is used as known to persons skilled in the art and is defined as a combination, wherein finerenone and pecavaptan are present in more than one unit or dosage form. It is possible for the components of the non-fixed combination or kit-of-parts to be administered separately, sequentially, simultaneously, concurrently or chronologically staggered. In one embodiment of a non-fixed combination or kit-of-parts finerenone and pecavaptan are present separately. In one embodiment the non-fixed combination or kit-of-parts comprises a pharmaceutical composition comprising finerenone and a pharmaceutical composition comprising pecavaptan. In one embodiment of a non-fixed combination or kit-of-parts comprises a dosage form comprising finerenone and a dosage form comprising pecavaptan. The non-fixed combination or kit-of-parts is not limited to this exemplary embodiment(s). Various combinations of the dosage forms disclosed herein are suitable for the combination according to the invention.

The non-fixed combination or kit-of-parts is particularly advantageous if the separate components have to be administered in different dose forms or are administered in different dose intervals.

In one embodiment, the combination according to the invention is a combination pack.

In a "combination pack" active substances are included in separate dosage forms marketed in the same package. The combination pack is used to combine two or more dose forms to describe products that are packaged together but are administered separately, sequentially, simultaneously, concurrently or chronologically staggered as independent pharmaceutical products. A combination is different from a combined pharmaceutical dose form. In one embodiment, the combination pack comprises a first dosage form comprising finerenone and a second dosage form comprising pecavaptan. In one embodiment the combination pack comprises first oral dosage form comprising finerenone and a second oral dosage form comprising pecavaptan. The oral dosage form can be selected from the oral dosage form and in any combination described herein. In one embodiment the combination pack comprises a tablet comprising finerenone and a granulate for oral solution comprising pecavaptan or *vice versa.* In this embodiment the combination comprises a tablet as administrable dose form and oral solution as administrable dose form.

In one embodiment the combinations according to the invention, finerenone and pecavaptan are administered successively or separately. In the combinations according to the invention, the compounds are administered in at least two separate dosage forms.

In one embodiment, the separate dosage forms are identical. In one example of this embodiment, the combination comprises a first tablet comprising finerenone and a second tablet comprising pecavaptan (herein, the separate dosage form are identical as both dosage forms are tablets). This embodiment is not limited to the combination of two tablets. Further dosage forms, which can be used in this embodiment are disclosed below. This embodiment, wherein separate dosage forms are identical, can be used for example in a combined pharmaceutical dose form, non-fixed-combination, kit-of-parts and/or combination pack.

In one embodiment, the separate dosage forms are different. In one example of this embodiment, the combination comprises a tablet comprising finerenone and a capsule comprising pecavaptan (herein, the separate dosage form are different as the dosage forms differ from each other). This embodiment is not limited to the combination of a tablet and capsule. Further dosage forms, which can be used in this embodiment are disclosed below. This embodiment, wherein separate dosage forms are different, can be used for example in a combined pharmaceutical dose form, non-fixed-combination, kit-of-parts and/or combination pack.

The release of one or more agents of the combination can also be controlled, where appropriate, to provide the desired therapeutic activity when in a single dosage form, combination pack, kit-of-parts or when in separate independent dosage forms.

In one embodiment the combination according to the invention is administered one or more times per day. The combination according to the invention can be applied once daily (once daily administration). In one embodiment administration is performed via the oral route once or more time per day. The combination according to the invention can also comprise two separate dosage forms, wherein the respective dosage form is given at a different time point.

The present invention includes pharmaceutical compositions or dosage forms which are comprised of a pharmaceutically effective amount of pecavaptan and finerenone and pharmaceutically acceptable carrier and/or excipient. A "pharmaceutically effective amount" of finerenone or pecavaptan (active ingredients) is that amount which produces a result or exerts an influence on the particular condition being treated. A "pharmaceutically acceptable carrier" is any carrier which is relatively non-toxic and innocuous to a patient at concentrations consistent with effective activity of the active ingredient so that any side effects ascribable to the carrier do not vitiate the beneficial effects of the active ingredient. An "excipient" is used as known oin the art. It is a substance formulated alongside the active ingredient of a medication, included for e.g. the purpose of long-term stabilization, bulking up solid formulations that contain potent active ingredients in small amounts (thus often referred to as "bulking agents", "fillers", or "diluents"), or to confer a therapeutic enhancement on the active ingredient in the final dosage form, such as facilitating drug absorption, reducing viscosity, or enhancing solubility. Excipients can also be useful in the manufacturing process, to aid in the handling of the active substance concerns such as by facilitating powder flowability or non-stick properties, in addition to aiding in vitro stability such as prevention of denaturation or aggregation over the expected shelf life. The selection of appropriate excipients also depends upon the route of administration and the dosage form, as well as the active ingredient and other factors. Some excipients are described below.

Commonly used pharmaceutical ingredients which can be used as appropriate to formulate the composition for its intended route of administration include:
- acidifying agents (examples include but are not limited to acetic acid, citric acid, fumaric acid, hydrochloric acid, nitric acid);
- alkalinizing agents (examples include but are not limited to ammonia solution, ammonium carbonate, diethanolamine, monoethanolamine, potassium hydroxide, sodium borate, sodium carbonate, sodium hydroxide, triethanolamine, trolamine);
- adsorbents (examples include but are not limited to powdered cellulose and activated charcoal);
- aerosol propellants (examples include but are not limited to carbon dioxide, CCl₂F₂, F₂ClC-CClF₂ and CClF₃);
- air displacement agents (examples include but are not limited to nitrogen and argon);
- antifungal preservatives (examples include but are not limited to benzoic acid, butylparaben, ethylparaben, methylparaben, propylparaben, sodium benzoate);
- antimicrobial preservatives (examples include but are not limited to benzalkonium chloride, benzethonium chloride, benzyl alcohol, cetylpyridinium chloride, chlorobutanol, phenol, phenylethyl alcohol, phenylmercuric nitrate and thimerosal);
- antioxidants (examples include but are not limited to ascorbic acid, ascorbyl palmitate, butylated hydroxyanisole, butylated hydroxytoluene, hypophosphorus acid, monothioglycerol, propyl gallate, sodium ascorbate, sodium bisulfite, sodium formaldehyde sulfoxylate, sodium metabisulfite);
- binding materials (examples include but are not limited to block polymers, natural and synthetic rubber, polyacrylates, polyurethanes, silicones, polysiloxanes and styrene-butadiene copolymers);
- buffering agents (examples include but are not limited to potassium metaphosphate, dipotassium phosphate, sodium acetate, sodium citrate anhydrous and sodium citrate dihydrate)
- carrying agents (examples include but are not limited to acacia syrup, aromatic syrup, aromatic elixir, cherry syrup, cocoa syrup, orange syrup, syrup, corn oil, mineral oil, peanut oil, sesame oil, bacteriostatic sodium chloride injection and bacteriostatic water for injection);
- chelating agents (examples include but are not limited to edetate disodium and edetic acid);
- colorants (examples include but are not limited to FD&C Red No. 3, FD&C Red No. 20, FD&C Yellow No. 6, FD&C Blue No. 2, D&C Green No. 5, D&C Orange No. 5, D&C Red No. 8, caramel and ferric oxide red);
- clarifying agents (examples include but are not limited to bentonite);
- emulsifying agents (examples include but are not limited to acacia, cetomacrogol, cetyl alcohol, glyceryl monostearate, lecithin, sorbitan monooleate, polyoxyethylene 50 monostearate);
- encapsulating agents (examples include but are not limited to gelatin and cellulose acetate phthalate);
- flavorants (examples include but are not limited to anise oil, cinnamon oil, cocoa, menthol, orange oil, peppermint oil and vanillin);
- humectants (examples include but are not limited to glycerol, propylene glycol and sorbitol);
- levigating agents (examples include but are not limited to mineral oil and glycerin);
- oils (examples include but are not limited to arachis oil, mineral oil, olive oil, peanut oil, sesame oil and vegetable oil);
- ointment bases (examples include but are not limited to lanolin, hydrophilic ointment, polyethylene glycol ointment, petrolatum, hydrophilic petrolatum, white ointment, yellow ointment, and rose water ointment);
- penetration enhancers (transdermal delivery) (examples include but are not limited to monohydroxy or polyhydroxy alcohols, mono-or polyvalent alcohols, saturated or unsaturated fatty alcohols, saturated or unsaturated fatty esters, saturated or unsaturated dicarboxylic acids, essential oils, phosphatidyl derivatives, cephalin, terpenes, amides, ethers, ketones and ureas);
- plasticizers (examples include but are not limited to diethyl phthalate and glycerol);
- solvents (examples include but are not limited to ethanol, corn oil, cottonseed oil, glycerol, isopropanol, mineral oil, oleic acid, peanut oil, purified water, water for injection, sterile water for injection and sterile water for irrigation);
- stiffening agents (examples include but are not limited to cetyl alcohol, cetyl esters wax, microcrystalline wax, paraffin, stearyl alcohol, white wax and yellow wax);
- suppository bases (examples include but are not limited to cocoa butter and polyethylene glycols (mixtures));
- surfactants (examples include but are not limited to benzalkonium chloride, nonoxynol 10, oxtoxynol 9, polysorbate 80, sodium lauryl sulfate and sorbitan mono-palmitate);
- suspending agents (examples include but are not limited to agar, bentonite, carbomers, carboxymethylcellulose sodium, hydroxyethyl cellulose, hydroxypropyl cellulose, hydroxypropyl methylcellulose, kaolin, methylcellulose, tragacanth and veegum);
- sweetening agents (examples include but are not limited to aspartame, dextrose, glycerol, mannitol, propylene glycol, saccharin sodium, sorbitol and sucrose);
- tablet anti-adherents (examples include but are not limited to magnesium stearate and talc);
- tablet binders (examples include but are not limited to acacia, alginic acid, carboxymethylcellulose sodium, compressible sugar, ethylcellulose, gelatin, liquid glucose, methylcellulose, non-crosslinked polyvinyl pyrrolidone, and pregelatinized starch);
- tablet and capsule diluents (examples include but are not limited to dibasic calcium phosphate, kaolin, lactose, mannitol, microcrystalline cellulose, powdered cellulose, precipitated calcium carbonate, sodium carbonate, sodium phosphate, sorbitol and starch);
- tablet coating agents (examples include but are not limited to liquid glucose, hydroxyethyl cellulose, hydroxypropyl cellulose, hydroxypropyl methylcellulose, methylcellulose, ethylcellulose, cellulose acetate phthalate and shellac);
- tablet direct compression excipients (examples include but are not limited to dibasic calcium phosphate);
- tablet disintegrants (examples include but are not limited to alginic acid, carboxymethylcellulose calcium, microcrystalline cellulose, polacrillin potassium, cross-linked polyvinylpyrrolidone, sodium alginate, sodium starch glycollate and starch);
- tablet glidants (examples include but are not limited to colloidal silica, corn starch and talc);
- tablet lubricants (examples include but are not limited to calcium stearate, magnesium stearate, mineral oil, stearic acid and zinc stearate);
- tablet/capsule opaquants (examples include but are not limited to titanium dioxide);
- tablet polishing agents (examples include but are not limited to carnauba wax and white wax);
- thickening agents (examples include but are not limited to beeswax, cetyl alcohol and paraffin);
- tonicity agents (examples include but are not limited to dextrose and sodium chloride);
- viscosity increasing agents (examples include but are not limited to alginic acid, bentonite, carbomers, carboxymethylcellulose sodium, methylcellulose, polyvinyl pyrrolidone, sodium alginate and tragacanth); and
- wetting agents (examples include but are not limited to heptadecaethylene oxycetanol, lecithin, sorbitol monooleate, polyoxyethylene sorbitol monooleate, and polyoxyethylene stearate).
It is believed that one skilled in the art, utilizing the preceding information, can utilize the present invention to its fullest extent.

Combinations of the present invention can be administered in any form by any effective route, including, for example oral, parenteral, enteral, intravenous, intraperitoneal, topical, transdermal (for example using any standard patch), ophthalmic, nasally, local, non-oral, such as aerosal, inhalation, subcutaneous, intramuscular, buccal, sublingual, rectal, vaginal, intra-arterial, and intrathecal, etc. They can be administered alone, or in combination with any ingredient(s), active or inactive.

Combinations of the present invention can be converted in a known manner into the usual dosage forms, pharmaceutical dose form or pharmaceutical formulations, which can be liquid or solid formulations for example without limitation tablets, coated tablets, capsules, pills, powders, granules, elixirs, tinctures, solution, suspensions, syrups, solid and liquid aerosols and emulsions.

In one embodiment the combination according to the invention is administered via oral administration. In one embodiment the combination according to the invention is comprised in one dosage form for oral administration. For oral administration, the compounds can be formulated into solid or liquid can be prepared according to methods known to the art for the manufacture of pharmaceutical compositions. Examples of dosage forms for oral administration of finerenone or pecavaptan are described in WO 2008/104306 A1 (see WO 2008/104306 A1, section C) and WO 2016/071212 A1 (see WO 2016/071212 A1, section C) as well as in the Examples of the present application in section A.1, "Tablet comprising finerenone" below.

In one embodiment the combination according to the invention is comprised in one dosage form. In one embodiment the combination according to the invention consists of two separate dosage form. In one embodiment the combination according to the invention consists of two separate dosage form for oral administration.

In one embodiment the combination according to the invention is a capsule. In one embodiment the combination according to the invention is a tablet. In one embodiment the combination according to the invention consist of one single tablet comprising finerenone and pecavaptan. In one embodiment the combination according to the invention comprises at least two tablets, wherein one tablet comprises finerenone and the other tablet comprises pecavaptan. In one embodiment the combination according to the invention comprises at least two tablets, wherein one tablet comprises finerenone, but no pecavaptan, and the other tablet comprises pecavaptan, but no finerenone.

In another embodiment, finerenone and/or pecavaptan to the invention can be tableted with conventional tablet bases such as lactose, sucrose and corn starch in combination with binders such as acacia, corn starch or gelatine, disintegrating agents intended to assist the break-up and dissolution of the tablet following administration such as potato starch, alginic acid, corn starch, and guar gum, gum tragacanth, acacia, lubricants intended to improve the flow of tablet granulation and to prevent the adhesion of tablet material to the surfaces of the tablet dies and punches, for example talc, stearic acid, or magnesium, calcium or zinc stearate, dyes, coloring agents, and flavoring agents such as peppermint, oil of wintergreen, or cherry flavoring, intended to enhance the aesthetic qualities of the tablets and make them more acceptable to the patient. Suitable excipients for use in oral liquid dosage forms include dicalcium phosphate and diluents such as water and alcohols, for example, ethanol, benzyl alcohol, and polyethylene alcohols, either with or without the addition of a pharmaceutically acceptable surfactant, suspending agent or emulsifying agent. Various other materials can be present as coatings or to otherwise modify the physical form of the dosage form. For instance tablets, pills or capsules can be coated with shellac, sugar or both.

Dispersible powders and granules are suitable for the preparation of an aqueous suspension. They provide the active ingredient in admixture with a dispersing or wetting agent, a suspending agent and one or more preservatives. Suitable dispersing or wetting agents and suspending agents are exemplified by those already mentioned above. Additional excipients, for example those sweetening, flavoring and coloring agents described above, may also be present.

The pharmaceutical compositions of this invention may also be in the form of oil-in-water emulsions. The oily phase can be a vegetable oil such as liquid paraffin or a mixture of vegetable oils. Suitable emulsifying agents can be (1) naturally occurring gums such as gum acacia and gum tragacanth, (2) naturally occurring phosphatides such as soy bean and lecithin, (3) esters or partial esters derived from fatty acids and hexitol anhydrides, for example, sorbitan monooleate, (4) condensation products of said partial esters with ethylene oxide, for example, polyoxyethylene sorbitan monooleate. The emulsions may also contain sweetening and flavoring agents.

Oily suspensions can be formulated by suspending the active ingredient in a vegetable oil such as, for example, arachis oil, olive oil, sesame oil or coconut oil, or in a mineral oil such as liquid paraffin. The oily suspensions may contain a thickening agent such as, for example, beeswax, hard paraffin, or cetyl alcohol. The suspensions may also contain one or more preservatives, for example, ethyl or *n*-propyl p-hydroxybenzoate; one or more coloring agents; one or more flavoring agents; and one or more sweetening agents such as sucrose or saccharin.

Syrups and elixirs can be formulated with sweetening agents such as, for example, glycerol, propylene glycol, sorbitol or sucrose. Such formulations may also contain a demulcent, and preservative, such as methyl and propyl parabens and flavoring and coloring agents.

The pharmaceutical compositions of this invention may also be in the form of a dispersion. A "dispersion" is a system in which distributed particles of one material are dispersed in a continuous phase (sometimes called matrix) of another material. The two phases may be in the same or different states of matter. Dispersions are classified in a number of different ways, including how large the particles are in relation to the particles of the continuous phase, whether or not precipitation occurs, and the presence of Brownian motion. In general, dispersions of particles sufficiently large for sedimentation are called suspensions, while those of smaller particles are called colloids and solutions.

The pharmaceutical compositions of this invention may also be in the form of a solid dispersion. The solid dispersion can be a solid solution, glass solution, glass suspension, amorphous precipitation in a crystalline carrier, eutectic or monotecic, compound or complex formation and combinations thereof.

An aspect of the invention of particular interest is a pharmaceutical composition comprising a solid dispersion, wherein the matrix comprises a pharmaceutically acceptable carrier is a polymer, such as polyvinylpyrrolidone, vinylpyrrolidone/vinylacetate copolymer, polyalkylene glycol (i.e. polyethylene glycol), hydroxyalkyl cellulose (i.e. hydroxypropyl cellulose), hydroxyalkyl methyl cellulose (i.e. hydroxypropyl methyl cellulose), carboxymethyl cellulose, sodium carboxymethyl cellulose, ethyl cellulose, polymethacrylates, polyvinyl alcohol, polyvinyl acetate, vinyl alcohol/vinyl acetate copolymer, polyglycolized glycerides, xanthan gum, carrageenan, chitosan, chitin, poyldextrin, dextrin, starch and proteins.

Another aspect of the invention is a pharmaceutical composition comprising a solid dispersion, wherein the matrix comprises a sugar and/or sugar alcohol and/or cyclodextrin, for example sucrose, lactose, fructose, maltose, raffinose, sorbitol, lactitol, mannitol, maltitol, erythritol, inositol, trehalose, isomalt, inulin, maltodextrin, β-cyclodextrin, hydroxypropyl-β-cyclodextrin or sulfobutyl ether cyclodextrin.

Additional suitable carriers that are useful in the formation of the matrix of the solid dispersion include, but are not limited to alcohols, organic acids, organic bases, amino acids, phospholipids, waxes, salts, fatty acid esters, polyoxyethylene sorbitan fatty acid esters, and urea.

The solid dispersion of finerenone in the matrix may contain certain additional pharmaceutical acceptable ingredients.

The solid dispersion of the invention is prepared according to methods known to the art for the manufacture of solid dispersions, such as fusion/melt technology, hot melt extrusion, solvent evaporation (i.e. freeze drying, spray drying or layering of powders of granules), coprecipitation, supercritical fluid technology and electrostatic spinning method.

The compositions of the invention can also contain other conventional pharmaceutically acceptable compounding ingredients, generally referred to as carriers or diluents, as necessary or desired. Conventional procedures for preparing such compositions in appropriate dosage forms can be utilized.

The amount of the administered active ingredient can vary widely according to such considerations as the particular compound and dosage form employed, the mode and time of administration, the period of treatment, the age, sex, and general condition of the patient treated, the nature and extent of the condition treated, the rate of drug metabolism and excretion, the potential drug combinations and drug-drug interactions, and the like.

One embodiment according to the invention refers to the combination comprising finerenone in an amount of 5 to 80 mg. In one embodiment the combination according to the invention comprises finerone in an amount of 5 to 80 mg, 5 to 70 mg, 5 to 60 mg, 5 to 50 mg, 5 to 40 mg, 10 to 80 mg, 10 to 70 mg, 10 to 60 mg, 10 to 50 mg or 10 to 40 mg. In one embodiment the combination according to the invention comprises finerenone in an amount of 10 to 40 mg. In one embodiment the combination according to the invention comprises finerenone in an amount of 20 to 40 mg. In one embodiment the combination according to the invention comprises finerenone in an amount of 5, 10, 15, 20, 25, 30, 35, 40, 45, 50, 55, 60, 65, 70, 75 or 80 mg. In one embodiment the combination according to the invention comprises finerenone in an amount of 10 mg. In one embodiment the combination according to the invention comprises finerenone in an amount of 20 mg. In one embodiment the combination according to the invention comprises finerenone in an amount of 30 mg. In one embodiment the combination according to the invention comprises finerenone in an amount of 40 mg.

One embodiment according to the invention refers to the combination comprising pecavaptan in an amount of 5 to 90 mg. In one embodiment the combination according to the invention comprises pecavaptan in an amount of 5 to 90 mg, 5 to 80 mg, 5 to 70 mg, 5 to 60 mg, 5 to 50 mg, 5 to 40 mg, 5 to 30 mg 10 to 90 mg, 10 to 80 mg, 10 to 70 mg, 10 to 60 mg, 10 to 50 mg or 10 to 40 mg, 10 to 30 mg 15 to 90 mg, 15 to 80 mg, 15 to 70 mg, 15 to 60 mg, 15 to 50 mg, 15 to 40 mg or 15 to 30 mg. In one embodiment the combination according to the invention comprises pecavaptan in an amount of 15 to 60 mg. In one embodiment the combination according to the invention comprises pecavaptan in an amount of 15 to 60 mg. In one embodiment the combination according to the invention comprises pecavaptan in an amount of 5, 10, 15, 20, 25, 30, 35, 40, 45, 50, 55, 60, 65, 70, 75, 80, 85 or 90 mg. In one embodiment the combination according to the invention comprises pecavaptan in an amount of 10 mg. In one embodiment the combination according to the invention comprises pecavaptan in an amount of 15 mg. In one embodiment the combination according to the invention comprises pecavaptan in an amount of 20 mg. In one embodiment the combination according to the invention comprises pecavaptan in an amount of 25 mg. In one embodiment the combination according to the invention comprises pecavaptan in an amount of 30 mg. combination according to the invention can comprise pecavaptan in an amount of 35 mg. In one embodiment the combination according to the invention comprises pecavaptan in an amount of 40 mg. In one embodiment the combination according to the invention comprises pecavaptan in an amount of 50 mg. In one embodiment the combination according to the invention comprises pecavaptan in an amount of 60 mg. In one embodiment the combination according to the invention comprises pecavaptan in an amount of 65 mg. In one embodiment the combination according to the invention comprises pecavaptan in an amount of 70 mg. In one embodiment the combination according to the invention comprises pecavaptan in an amount of 80 mg.

In one embodiment the combination according to the invention comprises finerenone in an amount 5 to 80 mg and pecavaptan in an amount of 5 to 90 mg. In one embodiment the combination according to the invention comprises finerenone in an amount 10 to 40 mg and pecavaptan in an amount of 15 to 30 mg.

In one embodiment the combination according to the invention comprises finerenone in an amount 10 mg and pecavaptan in an amount of 30 mg. In one embodiment the combination according to the invention comprises finerenone in an amount 10 mg and pecavaptan in an amount of 15 mg. In one embodiment the combination according to the invention comprises finerenone in an amount 20 mg and pecavaptan in an amount of 30 mg.

In one embodiment the combination according to the invention comprises finerenone in an amount 20 mg and pecavaptan in an amount of 20 mg. In one embodiment the combination according to the invention comprises finerenone in an amount 20 mg and pecavaptan in an amount of 15 mg. In one embodiment the combination according to the invention comprises finerenone in an amount 20 mg and pecavaptan in an amount of 10 mg.

In one embodiment the combination according to the invention comprises finerenone in an amount 30 mg and pecavaptan in an amount of 30 mg. In one embodiment the combination according to the invention comprises finerenone in an amount 30 mg and pecavaptan in an amount of 20 mg. In one embodiment the combination according to the invention comprises finerenone in an amount 30 mg and pecavaptan in an amount of 15 mg. In one embodiment the combination according to the invention comprises finerenone in an amount 30 mg and pecavaptan in an amount of 10 mg.

In one embodiment the combination according to the invention comprises finerenone in an amount 40 mg and pecavaptan in an amount of 30 mg. In one embodiment the combination according to the invention comprises finerenone in an amount 40 mg and pecavaptan in an amount of 20 mg. In one embodiment the combination according to the invention comprises finerenone in an amount 40 mg and pecavaptan in an amount of 15 mg. In one embodiment the combination according to the invention comprises finerenone in an amount 40 mg and pecavaptan in an amount of 10 mg.

Nevertheless, it may in some cases be advantageous to deviate from the amounts specified, depending on body weight, individual behaviour towards the active ingredient, type of preparation and time or interval over which the administration is affected. For instance, less than the aforementioned minimum amounts can be sufficient in some cases, while the upper limit specified has to be exceeded in other cases.

In the case of administration of relatively large amounts, it can be advisable to divide these into several individual doses over the day.

The relative ratios of each compound in the combination can also be selected based on their respective mechanisms of action and the disease biology. The relative ratios of each compound can vary widely.

The present invention also relates to a method for using the combination and compositions thereof, to treat cardiovascular disorders and/or of renal and cardiorenal disorders.

The diseases can be characterized by chronic sodium retention, such as chronic heart and kidney failure. This method comprises administering to a mammal in need thereof, including a human, an amount of the combination, which is effective to treat the disorder.

In one embodiment the combination according to the invention is used in the treatment and/or prevention of heart failure and/or chronic kidney diseases.

The combination according to the invention is suitable for the prophylaxis and/or treatment of various disorders and disease-related states, in particular for the treatment and/or prophylaxis of
- cardiovascular disorders such as congestive heart failure, acute heart failure, chronic heart failure, worsening chronic heart failure, hospitalization for heart failure, heart failure with preserved ejection fraction (HFpEF), heart failure with mid-range ejection fraction (HFmrEF) or heart failure with reduced ejection fraction (HFrEF);
- renal and cardiorenal disorders such as chronic kidney disease (CKD), diabetic and hypertensive kidney disease, cardiorenal syndrome, nephrotic syndrome;
- hepatorenal syndrome, renal hypoperfusion, intradialytic hypotension, obstructive uropathy, glomerulopathies, IgA nephropathy, glomerulonephritis, glomerulosclerosis, tubulointerstitial diseases, nephropathic diseases such as primary and congenital kidney disease, nephritis, Alport syndrome, kidney inflammation, immunological kidney diseases, kidney transplant rejection, immune complex-induced kidney diseases, nephropathy induced by toxic substances, contrast medium-induced nephropathy; minimal change glomerulonephritis (lipoid), focal segmental glomerulosclerosis (FSGS), amyloidosis, renal cysts, hypertensive nephrosclerosis and nephrotic syndrome (which can be characterized diagnostically, for example, by abnormally reduced creatinine and/or water excretion, abnormally increased blood concentrations of urea, nitrogen, potassium and/or creatinine, altered urine osmolarity or urine volume, increased microalbuminuria, macroalbuminuria, lesions of glomeruli and arterioles, tubular dilatation, hyperphosphataemia and/or the need for dialysis), uraemia, anaemia, electrolyte disturbances (for example hyperkalaemia, hyponatraemia), disturbances in bone and carbohydrate metabolism, polycystic kidney disease (PCKD) and of the syndrome of inadequate ADH secretion (SIADH);
- edema, pulmonary edema, cerebral edema, renal edema and heart failure-related edema;
- cirrhosis;
- NASH (non-alcoholic steatohepatitis);
- arterial hypertension, resistant hypertension, pulmonary hypertension;
- cardiovascular disorders such as hypertension, left ventricular dysfunction, hypertrophic cardiomyopathy, diabetic cardiomyopathy, supraventricular arrythmias, ventricular arrythmias, atrial fibrillation, atrial flutter;
- cardiovascular disorders such as stable angina pectoris, unstable angina pectoris, myocardial infarction and sequelae thereof, aneurysms, detrimental vascular remodelling, atherosclerosis, atrial fibrillation, stroke;
- shock such as cardiogenic shock, septic shock and anaphylactic shock;
- hypertensive kidney disease, peripheral arterial disease (PAD) including claudication and including critical limb ischemia, coronary microvascular dysfunction (CMD) including CMD type 1-4, primary and secondary Raynaud's phenomenon, microcirculation disturbances, peripheral and autonomic neuropathies, diabetic microangiopathies, diabetic retinopathy, diabetic limb ulcers, gangrene, CREST syndrome, erythematous disorders, rheumatic diseases, for promoting wound healing, inflammatory diseases, asthmatic diseases, chronic obstructive pulmonary disease (COPD), acute respiratory distress syndrome (ARDS), acute lung injury (ALI), alpha-1-antitrypsin deficiency (AATD), pulmonary fibrosis, pulmonary emphysema (for example smoking-induced pulmonary emphysema) and cystic fibrosis (CF);
- lung disorders and cardiopulmonary disorders such as pulmonary hypertension, disorders of the central nervous system;
- fibrotic disorders and other disease manifestations (for example end organ damage affecting brain, kidney or heart);
- multiple insults such as ischemia-reperfusion injury, radiocontrast administration, cardiopulmonary bypass surgery, shock and sepsis.

In one embodiment the combination according to the invention is used in the treatment and/or prevention of heart failure (congestive, acute, worsening and chronic, independent of ejection fraction), cardiorenal syndrome, CKD, diabetic and hypertensive kidney disease, nephrotic syndrome, hepatorenal syndrome, edema, cirrhosis, NASH (non-alcoholic steatohepatitis) and/or fibrotic disorders.

In one embodiment the combination according to the invention can be used for the prophylaxis and/or treatment of heart failure (independent of ejection fraction) and/or chronic kidney disease.

In one embodiment the combination according to the invention is used in the treatment and/or prevention of cardiovascular disorders such as congestive heart failure, acute heart failure, chronic heart failure, worsening chronic heart failure, hospitalization for heart failure, heart failure with preserved ejection fraction (HFpEF), heart failure with mid-range ejection fraction (HFmrEF) or heart failure with reduced ejection fraction (HFrEF).

In one embodiment the combination according to the invention is used in the treatment and/or prevention of renal and cardiorenal disorders such as chronic kidney disease (CKD), diabetic and hypertensive kidney disease, cardiorenal syndrome, nephrotic syndrome, hepatorenal syndrome, renal hypoperfusion, intradialytic hypotension, obstructive uropathy, glomerulopathies, IgA nephropathy, glomerulonephritis, glomerulosclerosis, tubulointerstitial diseases, nephropathic diseases such as primary and congenital kidney disease, nephritis, Alport syndrome, kidney inflammation, immunological kidney diseases, kidney transplant rejection, immune complex-induced kidney diseases, nephropathy induced by toxic substances, contrast medium-induced nephropathy; minimal change glomerulonephritis (lipoid), focal segmental glomerulosclerosis (FSGS), amyloidosis, renal cysts, hypertensive nephrosclerosis and nephrotic syndrome (which can be characterized diagnostically, for example, by abnormally reduced creatinine and/or water excretion, abnormally increased blood concentrations of urea, nitrogen, potassium and/or creatinine, altered urine osmolarity or urine volume, increased microalbuminuria, macroalbuminuria, lesions of glomeruli and arterioles, tubular dilatation, hyperphosphataemia and/or the need for dialysis), uraemia, anaemia, electrolyte disturbances (for example hyperkalaemia, hyponatraemia, disturbances in bone and carbohydrate metabolism, polycystic kidney disease (PCKD) and/or of the syndrome of inadequate ADH secretion (SIADH).

In one embodiment the combination according to the invention is used in the treatment and/or prevention of edema, pulmonary edema, cerebral edema, renal edema and/or heart failure-related edema.

In one embodiment the combination according to the invention is used in the treatment and/or prevention of cirrhosis and/or NASH (non-alcoholic steatohepatitis).

In one embodiment the combination according to the invention is used in the treatment and/or prevention of arterial hypertension, resistant hypertension and/or pulmonary hypertension.

In one embodiment the combination according to the invention is used in the treatment and/or prophylaxis of diseases characterized by sodium retention. These diseases are for example
- cardiovascular disorders such as congestive heart failure, acute heart failure, chronic heart failure, worsening chronic heart failure, hospitalization for heart failure, heart failure with preserved ejection fraction (HFpEF), heart failure with mid-range ejection fraction (HFmrEF) or heart failure with reduced ejection fraction (HFrEF);
- renal and cardiorenal disorders such as chronic kidney disease (CKD), diabetic and hypertensive kidney disease, cardiorenal syndrome, nephrotic syndrome;
- edema, pulmonary edema, cerebral edema, renal edema and heart failure-related edema;
- cirrhosis; and/or
- arterial hypertension, resistant hypertension, pulmonary hypertension.

The present invention further provides the use of the combination of the invention for the preparation of a pharmaceutical compositions for the treatment of the aforesaid diseases. Sometimes herein, the term "disorder" and disease" are used as synonyms.

The present invention further provides for the use of the combinations of the invention for production of a medicament for the treatment and/or prevention of the aforesaid diseases.

In one embodiment the combination according to the invention for production of a medicament can be used in the treatment of the aforesaid diseases.

Based upon standard laboratory techniques known to evaluate compounds useful for the treatment of cardiovascular diseases and/or of renal and/or cardiorenal diseases, by standard tests and by standard pharmacological assays for the determination of treatment of the conditions identified above in mammals, and by comparison of these results with the results of known medicaments that are used to treat these conditions, the effective dosage of the finerenone and/or pecavaptan can readily be determined for treatment of each desired indication. The amount of the active ingredient to be administered in the treatment of one of these conditions can vary widely according to such considerations as the particular compound and dosage form employed, the mode of administration, the period of treatment, the age and sex of the patient treated, and the nature and extent of the condition treated.

### Clauses

The following clauses form part of the disclosure and describe further embodiments according to the invention:
1. A combination comprising finerenone or a hydrate, solvate, pharmaceutically acceptable salt thereof, or a polymorph thereof, and pecavaptan, or a hydrate, solvate, pharmaceutically acceptable salt thereof, or a polymorph thereof.
2. The combination according to clause 1, wherein the combination is a fixed combination.
3. The combination according to any one of the preceding clauses, wherein the combination consists of a single dosage form.
4. The combination according to any one of the preceding clauses, wherein the combination consists of two separate dosage forms.
5. The combination according to clause 1 or 4, wherein the combination comprises the components:
   a. one dosage form comprising finerenone or a hydrate, solvate, pharmaceutically acceptable salt thereof, or a polymorph thereof, and
   b. one dosage form comprising pecavaptan or a hydrate, solvate, pharmaceutically acceptable salt thereof, or a polymorph thereof.
6. The combination according to clause 5, wherein the components a. and b. are administered separately, sequentially, simultaneously, concurrently or chronologically staggered.
7. The combination according to any one of the preceding clauses 1 to 6, wherein the combination is a combination pack or is part of a combination pack.
8. The combination according to any one of the preceding clauses 1 to 7, wherein the combination is a kit-of-parts or non-fixed combination.
9. The combination according to any one of the preceding clauses 1 to 8, wherein the combination is a dosage form for oral administration.
10. The combination according to any one of the preceding clauses 1 to 9, wherein the dosage form or one of the dosage forms or both of the dosage forms is a tablet.
11. The combination according to any one of the preceding clauses 1 to 9, wherein the dosage form or one of the dosage forms or both of the dosage forms is a capsule.
12. The combination according to any one of the preceding clauses 1 to 10, wherein the dosage form or one of the dosage forms or both of the dosage forms is a granulate.
13. The combination according to any one of the preceding clauses 1 to 12, wherein the combination comprises finerenone or a hydrate, solvate, pharmaceutically acceptable salt thereof, or a polymorph thereof, in an amount of 5 to 80 mg,.
14. The combination according to any one of the preceding clauses 1 to 13, wherein the combination comprises finerenone or a hydrate, solvate, pharmaceutically acceptable salt thereof, or a polymorph thereof, in an amount of 5 to 80 mg, in particular in an amount of 5 to 70 mg, 5 to 60 mg, 5 to 50 mg, 5 to 40 mg, 10 to 80 mg, 10 to 70 mg, 10 to 60 mg, 10 to 50 mg or 10 to 40 mg.
15. The combination according to any one of the preceding clauses 1 to 14, wherein the combination comprises finerenone or a hydrate, solvate, pharmaceutically acceptable salt thereof, or a polymorph thereof, in an amount of 5 to 70 mg, 5 to 60 mg, 5 to 50 mg, 5 to 40 mg, 10 to 80 mg, 10 to 70 mg, 10 to 60 mg, 10 to 50 mg or 10 to 40 mg.
16. The combination according to any one of the preceding clauses 1 to 15, wherein the combination comprises finerenone or a hydrate, solvate, pharmaceutically acceptable salt thereof, or a polymorph thereof, in an amount 20 to 40 mg.
17. The combination according to any one of the preceding clauses 1 to 16, wherein the combination comprises finerenone or a hydrate, solvate, pharmaceutically acceptable salt thereof, or a polymorph thereof, in an amount of 40 mg, 30 mg, 20 mg or 10 mg.
18. The combination according to any one of the preceding clauses 1 to 17, wherein the combination comprises finerenone or a hydrate, solvate, pharmaceutically acceptable salt thereof, or a polymorph thereof, in an amount of 40 mg.
19. The combination according to any one of the preceding clauses 1 to 18, wherein the combination comprises finerenone or a hydrate, solvate, pharmaceutically acceptable salt thereof, or a polymorph thereof, in an amount of 30 mg.
20. The combination according to any one of the preceding clauses 1 to 20, wherein the combination comprises finerenone or a hydrate, solvate, pharmaceutically acceptable salt thereof, or a polymorph thereof, in an amount of 20 mg.
21. The combination according to any one of the preceding clauses 1 to 20, wherein the combination comprises finerenone or a hydrate, solvate, pharmaceutically acceptable salt thereof, or a polymorph thereof, in an amount of 15 mg.
22. The combination according to any one of the preceding clauses 1 to 21, wherein the combination comprises finerenone or a hydrate, solvate, pharmaceutically acceptable salt thereof, or a polymorph thereof, in an amount of 10 mg.
23. The combination according to any one of the preceding clauses 1 to 22, wherein the combination comprises pecavaptan or a hydrate, solvate, pharmaceutically acceptable salt thereof, or a polymorph thereof, in an amount of 5 to 90 mg.
24. The combination according to any one of the preceding clauses 1 to 23, wherein the combination comprises pecavaptan or a hydrate, solvate, pharmaceutically acceptable salt thereof, or a polymorph thereof, in an amount of 5 to 80 mg, 5 to 70 mg, 5 to 60 mg, 5 to 50 mg, 5 to 40 mg, 5 to 30 mg 10 to 90 mg, 10 to 80 mg, 10 to 70 mg, 10 to 60 mg, 10 to 50 mg or 10 to 40 mg, 10 to 30 mg 15 to 90 mg, 15 to 80 mg, 15 to 70 mg, 15 to 60 mg, 15 to 50 mg, 15 to 40 mg or 15 to 30 mg.
25. The combination according to any one of the preceding clauses 1 to 24, wherein the combination comprises pecavaptan or a hydrate, solvate, pharmaceutically acceptable salt thereof, or a polymorph thereof, in an amount of 30 mg, 25 mg, 20 mg or 15 mg.
26. The combination according to any one of the preceding clauses 1 to 25, wherein the combination comprises pecavaptan or a hydrate, solvate, pharmaceutically acceptable salt thereof, or a polymorph thereof, in an amount of 30 mg.
27. The combination according to any one of the preceding clauses 1 to 26, wherein the combination comprises pecavaptan or a hydrate, solvate, pharmaceutically acceptable salt thereof, or a polymorph thereof, in an amount of 20 mg.
28. The combination according to any one of the preceding clauses 1 to 27, wherein the combination comprises pecavaptan or a hydrate, solvate, pharmaceutically acceptable salt thereof, or a polymorph thereof, in an amount of 15 mg.
29. The combination according to any one of the preceding clauses 1 to 28, wherein the combination comprises pecavaptan or a hydrate, solvate, pharmaceutically acceptable salt thereof, or a polymorph thereof, in an amount of 10 mg.
30. The combination according to any one of the preceding clauses 1 to 29, wherein the combination comprises finerenone or a hydrate, solvate, pharmaceutically acceptable salt thereof, or a polymorph thereof, in an amount 10 to 40 mg and pecavaptan in an amount of 15 to 30 mg.
31. The combination according to any one of the preceding clauses 1 to 30, wherein the combination comprises finerenone or a hydrate, solvate, pharmaceutically acceptable salt thereof, or a polymorph thereof, in an amount 40 mg and pecavaptan or a hydrate, solvate, pharmaceutically acceptable salt thereof, or a polymorph thereof, in an amount of 30 mg.
32. The combination according to any one of the preceding clauses 1 to 31, wherein the combination comprises finerenone or a hydrate, solvate, pharmaceutically acceptable salt thereof, or a polymorph thereof, in an amount 40 mg and pecavaptan or a hydrate, solvate, pharmaceutically acceptable salt thereof, or a polymorph thereof, in an amount of 15 mg.
33. The combination according to any one of the preceding clauses 1 to 32, wherein the combination comprises finerenone or a hydrate, solvate, pharmaceutically acceptable salt thereof, or a polymorph thereof, in an amount 20 mg and pecavaptan or a hydrate, solvate, pharmaceutically acceptable salt thereof, or a polymorph thereof, in an amount of 30 mg.
34. The combination according to any one of the preceding clauses 1 to 33, wherein the combination comprises finerenone or a hydrate, solvate, pharmaceutically acceptable salt thereof, or a polymorph thereof, in an amount 20 mg and pecavaptan or a hydrate, solvate, pharmaceutically acceptable salt thereof, or a polymorph thereof, in an amount of 15 mg.
35. The combination according to any one of the preceding clauses1 to 34, wherein the combination comprises finerenone or a hydrate, solvate, pharmaceutically acceptable salt thereof, or a polymorph thereof, in an amount 10 mg and pecavaptan or a hydrate, solvate, pharmaceutically acceptable salt thereof, or a polymorph thereof, in an amount of 30 mg.
36. The combination according to any one of the preceding clauses1 to 35, wherein the combination comprises finerenone or a hydrate, solvate, pharmaceutically acceptable salt thereof, or a polymorph thereof, in an amount 10 mg and pecavaptan or a hydrate, solvate, pharmaceutically acceptable salt thereof, or a polymorph thereof, in an amount of 15 mg.
37. The combination according to any one of the preceding clauses for once daily application.
38. Medicament comprising the combination according to any one of the preceding clauses 1 to 37.
39. Medicament according clause 28 comprising one or more inert, nontoxic, pharmaceutically suitable excipients.
40. The combination according to any one of clauses 1 to 37 or the medicament according to any one of clauses 38 or 39 for use as medicament.
41. The combination according to clause 40 for use as medicament for treating and/or preventing diseases.
42. The combination according to any one of clauses 1 to 37 or the medicament according to any one of clauses 38 or 39 for use as medicament for treating and/or preventing diseases, wherein the diseases is selected from
   - cardiovascular disorders such as congestive heart failure, acute heart failure, chronic heart failure, worsening chronic heart failure, hospitalization for heart failure, heart failure with preserved ejection fraction (HFpEF), heart failure with mid-range ejection fraction (HFmrEF) or heart failure with reduced ejection fraction (HFrEF);
   - renal and cardiorenal disorders such as chronic kidney disease (CKD), diabetic and hypertensive kidney disease, cardiorenal syndrome, nephrotic syndrome, hepatorenal syndrome, renal hypoperfusion, intradialytic hypotension, obstructive uropathy, glomerulopathies, IgA nephropathy, glomerulonephritis, glomerulosclerosis, tubulointerstitial diseases, nephropathic diseases such as primary and congenital kidney disease, nephritis, Alport syndrome, kidney inflammation, immunological kidney diseases, kidney transplant rejection, immune complex-induced kidney diseases, nephropathy induced by toxic substances, contrast medium-induced nephropathy; minimal change glomerulonephritis (lipoid), focal segmental glomerulosclerosis (FSGS), amyloidosis, renal cysts, hypertensive nephrosclerosis and nephrotic syndrome (which can be characterized diagnostically, for example, by abnormally reduced creatinine and/or water excretion, abnormally increased blood concentrations of urea, nitrogen, potassium and/or creatinine, altered urine osmolarity or urine volume, increased microalbuminuria, macroalbuminuria, lesions of glomeruli and arterioles, tubular dilatation, hyperphosphataemia and/or the need for dialysis), uraemia, anaemia, electrolyte disturbances (for example hyperkalaemia, hyponatraemia, disturbances in bone and carbohydrate metabolism, polycystic kidney disease (PCKD) and of the syndrome of inadequate ADH secretion (SIADH);
   - edema, pulmonary edema, cerebral edema, renal edema and heart failure-related edema;
   - cirrhosis;
   - NASH (non-alcoholic steatohepatitis);
   - arterial hypertension, resistant hypertension, pulmonary hypertension;
   - cardiovascular disorders such as hypertension, left ventricular dysfunction, hypertrophic cardiomyopathy, diabetic cardiomyopathy, supraventricular arrythmias, ventricular arrythmias, atrial fibrillation, atrial flutter,
   - cardiovascular disorders such as stable angina pectoris, unstable angina pectoris, myocardial infarction and sequelae thereof, aneurysms, detrimental vascular remodelling, atherosclerosis, atrial fibrillation, stroke;
   - shock such as cardiogenic shock, septic shock and anaphylactic shock;
   - hypertensive kidney disease, peripheral arterial disease (PAD) including claudication and including critical limb ischemia, coronary microvascular dysfunction (CMD) including CMD type 1-4, primary and secondary Raynaud's phenomenon, microcirculation disturbances, peripheral and autonomic neuropathies, diabetic microangiopathies, diabetic retinopathy, diabetic limb ulcers, gangrene, CREST syndrome, erythematous disorders, rheumatic diseases, for promoting wound healing, inflammatory diseases, asthmatic diseases, chronic obstructive pulmonary disease (COPD), acute respiratory distress syndrome (ARDS), acute lung injury (ALI), alpha-1-antitrypsin deficiency (AATD), pulmonary fibrosis, pulmonary emphysema (for example smoking-induced pulmonary emphysema) and cystic fibrosis (CF);
   - lung disorders and cardiopulmonary disorders such as pulmonary hypertension, disorders of the central nervous system;
   - fibrotic disorders and other disease manifestations (for example end organ damage affecting brain, kidney or heart);
   - multiple insults such as ischemia-reperfusion injury, radiocontrast administration, cardiopulmonary bypass surgery, shock and sepsis.
43. The combination for use according any one of clause 41 or 42, wherein the diseases is selected from heart failure (congestive, acute, worsening and chronic, independent of ejection fraction), cardiorenal syndrome, CKD, diabetic and hypertensive kidney disease, nephrotic syndrome, hepatorenal syndrome, edema, cirrhosis, NASH (non-alcoholic steatohepatitis) and/or fibrotic disorders.
44. The combination for use according any one of clause 41 or 42, wherein the diseases is selected from heart failure (independent of ejection fraction) and/or chronic kidney disease.
45. The combination for use according any one of clause 41 or 42, wherein the diseases is selected from cardiovascular disorders such as congestive heart failure, acute heart failure, chronic heart failure, worsening chronic heart failure, hospitalization for heart failure, heart failure with preserved ejection fraction (HFpEF), heart failure with mid-range ejection fraction (HFmrEF) or heart failure with reduced ejection fraction (HFrEF).
46. The combination for use according any one of clause 41 or 42, wherein the diseases is selected from renal and cardiorenal disorders such as chronic kidney disease (CKD), diabetic and hypertensive kidney disease, cardiorenal syndrome, nephrotic syndrome, hepatorenal syndrome, renal hypoperfusion, intradialytic hypotension, obstructive uropathy, glomerulopathies, IgA nephropathy, glomerulonephritis, glomerulosclerosis, tubulointerstitial diseases, nephropathic diseases such as primary and congenital kidney disease, nephritis, Alport syndrome, kidney inflammation, immunological kidney diseases, kidney transplant rejection, immune complex-induced kidney diseases, nephropathy induced by toxic substances, contrast medium-induced nephropathy; minimal change glomerulonephritis (lipoid), focal segmental glomerulosclerosis (FSGS), amyloidosis, renal cysts, hypertensive nephrosclerosis and nephrotic syndrome (which can be characterized diagnostically, for example, by abnormally reduced creatinine and/or water excretion, abnormally increased blood concentrations of urea, nitrogen, potassium and/or creatinine, altered urine osmolarity or urine volume, increased microalbuminuria, macroalbuminuria, lesions of glomeruli and arterioles, tubular dilatation, hyperphosphataemia and/or the need for dialysis), uraemia, anaemia, electrolyte disturbances (for example hyperkalaemia, hyponatraemia, disturbances in bone and carbohydrate metabolism, polycystic kidney disease (PCKD) and/or of the syndrome of inadequate ADH secretion (SIADH).
47. The combination for use according any one of clause 41 or 42, wherein the diseases is selected from edema, pulmonary edema, cerebral edema, renal edema and/or heart failure-related edema.
48. The combination for use according any one of clause 41 or 42, wherein the diseases is selected from cirrhosis and/or NASH (non-alcoholic steatohepatitis).
49. The combination for use according any one of clause 41 or 42, wherein the diseases is selected from arterial hypertension, resistant hypertension and/or pulmonary hypertension.
50. A method of treatment for preventing and/or treatment of diseases in a subject in need thereof using the combination according to any one of clauses 1 to 37 or medicament according to any one of clauses 38 or 39.
51. The method of treatment according to clause 50, wherein the diseases is selected from the diseases listed in any one of clauses 42 to 49
52. A method of treating and/or preventing diseases in a subject in need thereof comprising administering effective amounts of a mineralocorticoid receptor antagonist and a vasopressin receptor antagonist.
53. A method of treating and/or preventing disease in a subject in need thereof according to clause 52 comprising administering effective amounts of finerenone or a hydrate, solvate, pharmaceutically acceptable salt thereof, or a polymorph thereof and pecavaptan, or a hydrate, solvate, pharmaceutically acceptable salt thereof, or a polymorph thereof.
54. Use of the the combination according to any one of clauses 1 to 37 or medicament according to any one of clauses 38 or 39 for p production of a medicament for the treatment and/or prophylaxis of diseases.
55. The use according to clause 54, wherein the diseases is selected from the diseases listed in any one of clauses 42 to 49.

### Description of the figure

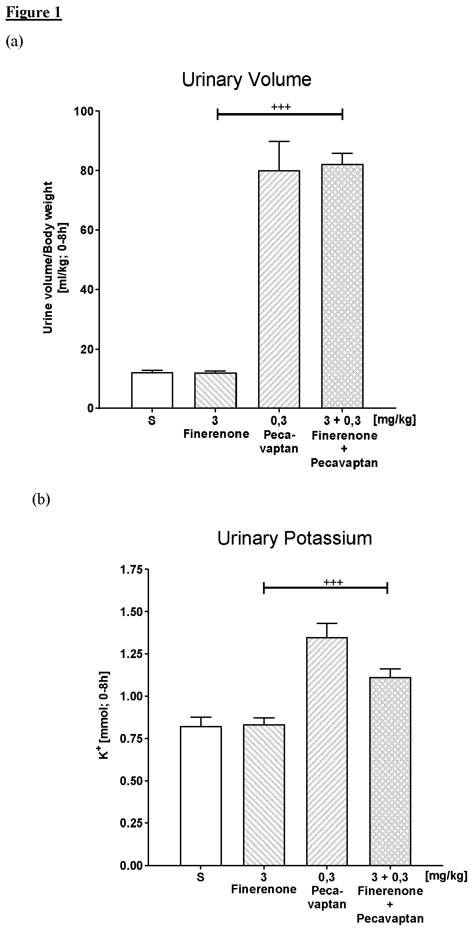

**Figure 1a****:** Influence of the MR antagonist finerenone (group B), the dual vasopressin V1a / V2 receptor antagonist pecavaptan (group C), and their combination (group D) on urinary volume of awake Wistar rats with activated renin-angiotensin-aldosterone system, which were examined according to section B above, 'assessment of the physiological effectiveness' for 8 hours after oral administration of the substances in metabolic cages. n = 7-8 animals / per group. +++: p <0.005 vs. Group Combination. S means solvent (group A). 3 mg/kg Finerenone (Group B). 0,3 mg/kg Pecavaptan (group C). 3 mg/kg Finerenone + 0,3 mg/kg Pecavaptan (group D).
**Figure 1b****:** Influence of the MR antagonist finerenone (group B), the dual vasopressin V1a / V2 receptor antagonist pecavaptan (group C), and their combination (group D) on the urinary potassium concentration of awake Wistar rats with activated renin-angiotensin-aldosterone system, which were examined according to section B above, 'assessment of the physiological effectiveness' for 8 hours after oral administration of the substances in metabolic cages. n = 7-8 animals / per group. +++: p <0.005 vs. Group Combination. S means solvent (group A). 3 mg/kg Finerenone (Group

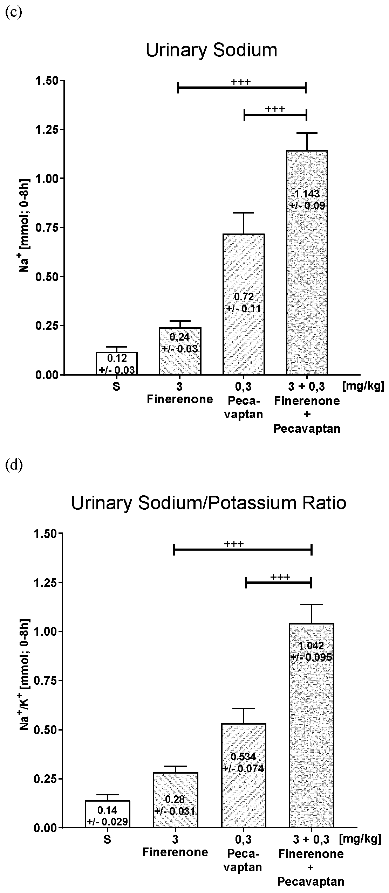

**Figure 1c****:** Influence of the MR antagonist finerenone (group B), the dual vasopressin V1a / V2 receptor antagonist pecavaptan (group C), and their combination (group D) on the urinary sodium concentration of awake Wistar rats with activated renin-angiotensin-aldosterone system, which were examined according to section B above, 'assessment of the physiological effectiveness' for 8 hours after oral administration of the substances in metabolic cages. n = 7-8 animals / per group. +++: p <0.005 vs. Group Combination. S means solvent (group A). 3 mg/kg Finerenone (Group
**Figure 1d****:** Influence of the MR antagonist finerenone (group B), the dual vasopressin V1a / V2 receptor antagonist pecavaptan (group C), and their combination (group D) on the resulting urinary sodium / potassium concentration ratio of awake Wistar rats with activated renin-angiotensin-aldosterone system, which were examined according to section B above, 'assessment of the physiological effectiveness' for 8 hours after oral administration of the substances in metabolic cages. n = 7-8 animals / per group. +++: p <0.005 vs. Group Combination. S means solvent (group A). 3 mg/kg Finerenone (Group

### Examples

### A - Pharmaceutical formulation/dosage form

### A-1: Tablet comprising finerenone (4S) - 4- (4-cyano-2-methoxyphenyl) -5-ethoxy-2,8-dimethyl-1,4-dihydro-1,6-naphthyridine-3-carboxamide of the formula (I)

A granulate solution of the compound of formula (I) in crystalline form in micronized form, hypromellose 5 cP, sodium lauryl sulfate in purified water was prepared. Microcrystalline cellulose, lactose monohydrate and croscarmellose sodium were mixed in a container or a fluidized bed granulator (premix). The premix and the granulate solution were granulated in the fluid-bed granulator. The lubricant magnesium stearate was added after the granules were dried and sieved. A ready-to-press mixture was thus produced. The ready-to-press mixture was pressed into tablets using a rotary tablet press.

A homogeneous coating suspension was made from hypromellose, talc, titanium dioxide, yellow iron oxide, red iron oxide and purified water. The coating suspension was sprayed onto the tablets in a suitable coating device.

**Table 1: Tablets (number 1 to 7) obtained by the process described above**

| Composition | | Tablets | | | | | | |
|---|---|---|---|---|---|---|---|---|
| | | 1 [mg] | 2 [mg] | 3 [mg] | 4 [mg] | 5 [mg] | 6 [mg] | 7 [mg] |
| | Finerenone, micronized | 1.25 | 2.50 | 5.00 | 7.50 | 10.00 | 15.00 | 20.00 |
| Excipients | Cellulose microcrystalline | 73.80 | 72.50 | 69.90 | 67.30 | 64.70 | 62.00 | 59.30 |
| | Croscarmellose sodium | 4.50 | 4.50 | 4.50 | 4.50 | 4.50 | 4.50 | 4.50 |
| | Hypromellose 5 cP | 4.50 | 4.50 | 4.50 | 4.50 | 4.50 | 4.50 | 4.50 |
| | Lactose monohydrate | 45.00 | 45.00 | 45.00 | 45.00 | 45.00 | 42.50 | 40.00 |
| | Magnesium stearate | 0.90 | 0.90 | 0.90 | 0.90 | 0.90 | 0.90 | 0.90 |
| | Sodium laurilsulfate | 0.05 | 0.10 | 0.20 | 0.30 | 0.40 | 0.60 | 0.80 |
| | Weight (uncoated tablet) | 130.00 | 130.00 | 130.00 | 130.00 | 130.00 | 130.00 | 130.00 |
| Film-coating | Hypromellose 5 cP | 3.0336 | 3.0336 | 3.0336 | 3.0336 | 3.0336 | 3.0336 | 3.0336 |
| | Titanium dioxide | 2.3196 | 2.3196 | 2.3196 | 2.3196 | 2.3196 | 2.3196 | 2.3196 |
| | Talcum | 0.6072 | 0.6072 | 0.6072 | 0.6072 | 0.6072 | 0.6072 | 0.6072 |
| | Iron oxide yellow | 0.0324 | 0.0324 | 0.0324 | 0.0324 | 0.0324 | 0.0324 | 0.0324 |
| | Iro oxide red | 0.0072 | 0.0072 | 0.0072 | 0.0072 | 0.0072 | 0.0072 | 0.0072 |
| | Weight (film-coating) | 6.0000 | 6.0000 | 6.0000 | 6.0000 | 6.0000 | 6.0000 | 6.0000 |
| | Weight (coated tablet) | 136.00 | 136.00 | 136.00 | 136.00 | 136.00 | 136.00 | 136.00 |

### B - Assessment of physiological effectiveness

### B-1 In vivo assay for detecting natriuretic activity in conscious rats with activated RAAS

Wistar rats (ca. 250-500 g body weight) were kept with free access to feed (Altromin) and drinking water. From approx. 72 hours before the start of the test, the animals received, instead of the normal feed, exclusively reduced-salt feed with a sodium chloride content of 0.02% Sodium Chloride (ssniff R/M-H, 10 mm with 0,02% Na, S0602-E081, ssniff Spezialdiäten GmbH, D-59494 Soest). This sodium-reduced feed causes an activation of the RAAS in the animals during the 3 days run-in phase and therefore mimics a neuroendocrine activation which is characteristic for cardiorenal diseases. During the test, the animals were housed singly in metabolic cages suitable for rats of this weight class (Tecniplast Deutschland GmbH, D-82383 Hohenpeissenberg) with free access to reduced-salt feed and drinking water for up to 24 hours. At the start of the test, the substance to be tested was administered by means of gavage in a volume of 2-3 ml/kg of body weight of a suitable solvent ('Adalat Placebo': 85.8% PEG400, 5.3% glycerol, 8.9% water). Control animals received only solvent ('Adalat Placebo' or solvent in table 2 below). Controls and substance tests were carried out in parallel on the same day. Control groups and substance-dose groups each consisted of 7 to 8 animals. During the test, the urine excreted by the animals was continuously collected in a receiver on the base of the cage. The urine volume per collection time was determined separately for each animal, and the concentration of the sodium and potassium ions excreted in the urine was measured by standard methods of flame photometry by a clinical-chemical analyzer system (ADVIA 2400, Siemens). The sodium/potassium ratio was calculated on the basis of the individual urinary sodium and potassium concentrations. The measurement intervals were typically the period up to 8 hours after the start of the test (day interval) or up to 24 hours after the start of the test (day and night interval).

### B-2 Comparison of monotherapy (finerenone or pecavaptan) with combination therapy (combined use of finerenone and pecavaptan)

In this comparison, four different administrations were performed:
Group A received the solvent (Adalat Placebo) only. This group serves as control group. Group B received finerenone only. It serves to detect the effect of the monotherapy with finerenone.
Group C received pecavaptan only. It serves to detect the effect of the monotherapy with pecavaptan. Group D received finerenone and pecavaptan in combination. It serves to detect the effect of the combined therapy with finerenone and pecavaptan (combination according to the invention). After the treatment, the urinary volume, urinary sodium and potassium concentrations were measured. Based on this measurement the sodium/potassium ratio was calculated. Table 2 summarizes the results.

**Table 2: Measured data (mean values ± standard deviation [SD]) of urinary volume, urinary potassium concentration (K+), urinary sodium concentration (Na+), and the resulting urinary sodium / potassium concentration ratio from awake Wistar rats with activated renin-angiotensin-aldosterone system (n = 7-8 animals per group), which were examined according to the 'assessment of the physiological efficacy' for 8 hours after oral administration of the substances in metabolic cages.**

| Group | Volume urine / body weight | Urinary K+ concentration [mmol] | Urinary Na+ concentration [mmol] | Urinary Na+/K+ concentration ratio |
|---|---|---|---|---|
| A: Solvent Mean value ± SD | 12.191± 0.606 | 0.83 ±0.05 | 0.12 ±0.03 | 0.140 ±0.029 |
| B: 3 mg/kg Finerenone (Monotherapy) Mean value ± SD | 12.069 ±0.563 | 0.84 ±0.04 | 0.24 ±0.03 | 0.283 ±0.031 |
| C: 0.3 mg/kg Pecavaptan (Monotherapy) Mean value ± SD | 80.070 ±9.661 | 1.35 ±0.08 | 0.72 ±0.11 | 0.534 ±0.074 |
| Combination group D: 3 mg/kg Finerenone + 0.3 mg/kg Pecavaptan (Combination) Mean value ± SD | 82.206 ±3.533 | 1.11 ±0.05 | 1.14 ±0.09 | 1.042 ±0.095 |
| | p<0.005 vs. group B | p<0.005 vs. group B | p<0.005 vs. group B and group C | p<0.005 vs. group B and group C |

The results are also depicted in Figure 1.
**Figure 1****:** Influence of the MR antagonist finerenone (group B), the dual vasopressin V1a / V2 receptor antagonist pecavaptan (group C), and their combination (group D) on
(a) urinary volume (figure 1a),
(b) the urinary potassium concentration (figure 1b),
(c) the urinary sodium concentration (figure 1c), and
(d) the resulting urinary sodium / potassium concentration ratio (figure Id)
of awake Wistar rats with activated renin-angiotensin-aldosterone system, which were examined according to section B above, 'assessment of the physiological effectiveness' for 8 hours after oral administration of the substances in metabolic cages. n = 7-8 animals / per group. +++: p <0.005 vs. Group Combination. S means solvent (group A). 3 mg/kg Finerenone (Group B). 0,3 mg/kg Pecavaptan (group C). 3 mg/kg Finerenone + 0,3 mg/kg Pecavaptan (group D).

From table 2 and figure 1 a to d the following can be concluded:
Group A, in which the solvent (Adalat Placebo) was administered, shows the physiological excretion of volume, potassium and sodium of conscious rats over 8 hours under RAAS activation. Group B shows that administration of 3 mg/kg of finerenone has no influence on urinary volume and urinary potassium, but causes an increase in urinary sodium and, thus, an increase of the urinary sodium/potassium ratio. Group C shows that administration of 0.3 mg/kg of pecavaptan increases urinary volume, potassium and sodium excretion. Since the relative sodium excretion is higher than the potassium excretion, the urinary sodium/potassium ratio is increased as well. Group D shows that administration of a combination of 3 mg/kg finerenone and 0.3 mg/kg of pecavaptan increases urinary volume, potassium and sodium excretion, with a resulting increase in the urinary sodium/potassium ratio. The increase in urinary volume and potassium is not statistically is approximately the same as the respective monotherapy with pecavaptan.. However, urinary sodium concentration as well as urinary sodium/potassium concentration ratio are statistically significant higher and, thus, more than the sum of the respective monotherapies. Therefore, the combination of finerenone and pecavaptan under typical conditions of activated RAAS leads to a significant natriuretic efficacy improvement over the sum of the monotherapies (cf. Groups B and C). This natriuretic efficacy improvement is a major clinical goal in the treatment of cardiovascular and/or cardiorenal diseases such as congestive heart failure and CKD.

## Claims

1. A pharmaceutical combination comprising finerenone or a hydrate, solvate, pharmaceutically acceptable salt thereof, or a polymorph thereof, and pecavaptan, or a hydrate, solvate, pharmaceutically acceptable salt thereof, or a polymorph thereof.

2. The combination according to claim 1, wherein the combination is selected from the group consisting of a fixed combination, a single dosage form, non-fixed combination, kit-of-parts and a combination pack.

3. The combination according to claim 1 or 2, wherein the components are administered separately, sequentially, simultaneously, concurrently or chronologically staggered.

4. The combination according to any one of claims 1 to 3, wherein the combination is a dosage form for oral administration.

5. The combination according to any one of claims 1 to 4, wherein the dosage form is a tablet.

6. The combination according to claim 5, wherein one tablet comprises finerenone or a hydrate, solvate, pharmaceutically acceptable salt thereof, or a polymorph thereof, and the other tablet comprises pecavaptan or a hydrate, solvate, pharmaceutically acceptable salt thereof, or a polymorph thereof.

7. The combination according to any one of claims 1 to 6, wherein the combination comprises finerenone or a hydrate, solvate, pharmaceutically acceptable salt thereof, or a polymorph thereof, in an amount of 5 to 80 mg.

8. The combination according to any one of claims 1 to 7, wherein the combination comprises pecavaptan or a hydrate, solvate, pharmaceutically acceptable salt thereof, or a polymorph thereof in an amount of 5 to 90 mg.

9. The combination according to any one of the preceding clauses, wherein the combination comprises finerenone or a hydrate, solvate, pharmaceutically acceptable salt thereof, or a polymorph thereof, in an amount of 40 mg, 30 mg, 20 mg or 10 mg and/or wherein the combination comprises pecavaptan or a hydrate, solvate, pharmaceutically acceptable salt thereof, or a polymorph thereof, in an amount of 30 mg, 25 mg, 20 mg or 15 mg.

10. The combination according to any one of claims 1 to 9 for once daily application.

11. Medicament comprising the combination according to any one of claims 1 to 9.

12. The combination according to any one of claims 1 to 9 for use as medicament.

13. The combination according to claim 12 for use as medicament for treating and/or preventing diseases.

14. The combination according to claim 12 or 13 for use as medicament for treating and/or preventing diseases, wherein the diseases is selected from
• cardiovascular disorders such as congestive heart failure, acute heart failure, chronic heart failure, worsening chronic heart failure, hospitalization for heart failure, heart failure with preserved ejection fraction (HFpEF), heart failure with mid-range ejection fraction (HFmrEF) or heart failure with reduced ejection fraction (HFrEF);
• renal and cardiorenal disorders such as chronic kidney disease (CKD), diabetic and hypertensive kidney disease, cardiorenal syndrome, nephrotic syndrome, hepatorenal syndrome, renal hypoperfusion, intradialytic hypotension, obstructive uropathy, glomerulopathies, IgA nephropathy, glomerulonephritis, glomerulosclerosis, tubulointerstitial diseases, nephropathic diseases such as primary and congenital kidney disease, nephritis, Alport syndrome, kidney inflammation, immunological kidney diseases, kidney transplant rejection, immune complex-induced kidney diseases, nephropathy induced by toxic substances, contrast medium-induced nephropathy; minimal change glomerulonephritis (lipoid), focal segmental glomerulosclerosis (FSGS), amyloidosis, renal cysts, hypertensive nephrosclerosis and nephrotic syndrome (which can be characterized diagnostically, for example, by abnormally reduced creatinine and/or water excretion, abnormally increased blood concentrations of urea, nitrogen, potassium and/or creatinine, altered urine osmolarity or urine volume, increased microalbuminuria, macroalbuminuria, lesions of glomeruli and arterioles, tubular dilatation, hyperphosphataemia and/or the need for dialysis), uraemia, anaemia, electrolyte disturbances (for example hyperkalaemia, hyponatraemia, disturbances in bone and carbohydrate metabolism, polycystic kidney disease (PCKD) and of the syndrome of inadequate ADH secretion (SIADH);
• edema, pulmonary edema, cerebral edema, renal edema and heart failure-related edema;
• cirrhosis;
• arterial hypertension, resistant hypertension, pulmonary hypertension;
• cardiovascular disorders such as hypertension, left ventricular dysfunction, hypertrophic cardiomyopathy, diabetic cardiomyopathy, supraventricular arrythmias, ventricular arrythmias, atrial fibrillation, atrial flutter,
• cardiovascular disorders such as stable angina pectoris, unstable angina pectoris, myocardial infarction and sequelae thereof, aneurysms, detrimental vascular remodelling, atherosclerosis, atrial fibrillation, stroke;
• shock such as cardiogenic shock, septic shock and anaphylactic shock;
• hypertensive kidney disease, peripheral arterial disease (PAD) including claudication and including critical limb ischemia, coronary microvascular dysfunction (CMD) including CMD type 1-4, primary and secondary Raynaud's phenomenon, microcirculation disturbances, peripheral and autonomic neuropathies, diabetic microangiopathies, diabetic retinopathy, diabetic limb ulcers, gangrene, CREST syndrome, erythematous disorders, rheumatic diseases, for promoting wound healing, inflammatory diseases, asthmatic diseases, chronic obstructive pulmonary disease (COPD), acute respiratory distress syndrome (ARDS), acute lung injury (ALI), alpha-1-antitrypsin deficiency (AATD), pulmonary fibrosis, pulmonary emphysema (for example smoking-induced pulmonary emphysema) and cystic fibrosis (CF);
• lung disorders and cardiopulmonary disorders such as pulmonary hypertension, disorders of the central nervous system;
• fibrotic disorders and other disease manifestations (for example end organ damage affecting brain, kidney or heart);
• multiple insults such as ischemia-reperfusion injury, radiocontrast administration, cardiopulmonary bypass surgery, shock and sepsis.

15. A method of treatment for preventing and/or treatment of diseases using the combination or medicament comprising the combination according to any one of claims 1 to 9.
